# EUROPEAN PATENT SPECIFICATION

(11) **EP 2 480 220 B1**
(45) Date of publication and mention of the grant of the patent: **19.11.2014**
(21) Application number: 10762626.9
(22) Date of filing: 22.09.2010
(51) Int. Cl.: A61K 9/51, A61K 9/08, A61K 31/352, A61P 31/18

(54) **Treatment and prevention of hiv infection**
Behandlung und Vorbeugung einer HIV-Infektion
Traitement et prévention de l'infection par VIH

(30) Priority: 22.09.2009 EP 09170916
(43) Date of publication of application: 01.08.2012
(73) Proprietor: Janssen R&D Ireland, Eastgate, Little Island, County Cork (IE)
(72) Inventor: BAERT, Lieven Elvire Colette, B-8200 Brugge 2 (BE); KRAUS, Guenter, B-2860 Sint-Katelijne-Waver (BE)
(74) Representative: Daelemans, Frank F.R.
(86) International application number: PCT/EP2010/063930
(87) International publication number: WO 2011/036159

(56) References cited:
- US-B1- 6 221 400
- FORD SUSAN L ET AL: "Single-dose safety and pharmacokinetics of brecanavir, a novel human immunodeficiency virus protease inhibitor", June 2006 (2006-06), ANTIMICROBIAL AGENTS AND CHEMOTHERAPY, VOL. 50, NR. 6, PAGE(S) 2201-2206, XP002568506, ISSN: 0066-4804 cited in the application page 2201, right-hand column, paragraph - page 2202, left-hand column, paragraph 1

## Description

### Field of the Invention

This invention relates to the long term treatment of HIV infection by intermittently administering a parenteral formulation comprising brecanavir at relatively long time intervals. This invention further concerns pharmaceutical compositions for parenteral administration, comprising micro- or nanoparticles of brecanavir, suspended in an aqueous pharmaceutically acceptable carrier, for the treatment and prophylaxis of HIV infection.

### Background of the Invention

The treatment of Human Immunodeficiency Virus (HIV) infection, known as cause of the acquired immunodeficiency syndrome (AIDS), remains a major medical challenge. HIV is able to evade immunological pressure, to adapt to a variety of cell types and growth conditions and to develop resistance against currently available drug therapies. The latter include nucleoside reverse transcriptase inhibitors (NRTIs), non-nucleoside reverse transcriptase inhibitors (NNRTIs), nucleotide reverse transcriptase inhibitors (NtRTIs), HIV-protease inhibitors (PIs) and the more recent fusion inhibitors (also known as entry inhibitors).

Although effective in suppressing HIV, each of these drugs, when used alone, is confronted with the emergence of resistant mutants. This led to the introduction of combination therapy of several anti-HIV agents usually having a different activity profile. In particular the introduction of "HAART" (Highly Active Anti-Retroviral Therapy) resulted in a remarkable improvement in anti-HIV therapy, leading to a large reduction in HIV-associated morbity and mortality. Current guidelines for anti-retroviral therapy recommend such triple combination therapy regimen even for initial treatment. However, none of the currently available drug therapies is capable of completely eradicating HIV. Even HAART can face the emergence of resistance, often due to no-adherence and non-persistence with anti-retroviral therapy. In these cases HAART can be made effective again by replacing one of its components by one of another class. If applied correctly, treatment with HAART combinations can suppress the virus for many years, up to decades, to a level where it no longer can cause the outbreak of AIDS.

One class of HIV drugs often used in HAART is that of the PIs, a number of which are currently on the market and several others are in various stages of development. A PI that has been in development is the compound [(3*R*,3a*S*,6a*R*)-2,3,3a,4,5,6a-hexahydro-furo[5,4-b]furan-3-yl] *N*-[(2*S*,3*R*)-4-(1,3-benzodioxol-5-ylsulfonyl-(2-methylpropyl)-amino)-3-hydroxy-1-[4-[(2-methyl-1,3-thiazol-4-yl)methoxy]phenyl]butan-2-yl]-carbamate also named *N-*(3*R*,3a*S*,6a*R*)-hexahydrofuro[2,3-b]furan-3-yl-oxycarbonyl-, (4*S*,5*R*)-4-[4-(2-methylthiazolo-4-methyloxy)-benzyl]-5-7-butyl-[(3,4-methylenedioxy-phenyl)sulfonyl]-aminomethyl-2,2-dimethyl-oxazolidine (CAS 313682-08-5), generically referred to as brecanavir. Although this compound showed pronounced activity against wild type HIV as well as against mutated variants, development was discontinued because of insurmountable issues regarding formulation.

Because of their pharmacokinetic properties and the need to keep plasma levels above a minimum level, most anti-HIV drugs require frequent administration of relatively high doses. The number and/or volume of dosage forms that need to be administered are commonly referred to as the "pill burden". A high pill burden is undesirable for many reasons, such as the frequency of intake, often combined with the inconvenience of having to swallow large dosage forms, as well as the need to store and transport a large number or volume of pills. A high pill burden increases the risk of patients not taking their entire dose, thereby failing to comply with the prescribed dosage regimen. As well as reducing the effectiveness of the treatment, this also leads to the emergence of viral resistance. The problems associated with a high pill burden are multiplied where a patient must take a combination of different anti-HIV agents.

Therefore, it would be desirable to provide HIV inhibitory therapy that reduces pill burden in that it involves the administration of dosage forms of relatively small size and additionally does not require frequent dosing. It would be attractive to provide anti-HIV therapy involving the administration of dosage forms at long time intervals such as two weeks or longer, or even one month or longer.

Oral administration of brecanavir without an agent that has a positive effect on drug metabolism and/or pharmacokinetics to improve bioavailability such as, for example, of ritonavir resulted in brecanavir exposures predicted to be insufficient to inhibit PI-resistant virus based on *in vitro* data. Co-administration of brecanavir with ritonavir, however, significantly increased the plasma brecanavir area under the concentration-time curve and maximum concentration, achieving brecanavir concentrations predicted to inhibit PI-resistant HIV (Antimicrob. Agents Chemother. 50 : 2201-2206). The compound brecanavir, its pharmacological activity as well as a number of procedures for its preparation have been described in WO 2000/076961. Co-administration of ritonavir not only adds to the pill burden, but ritonavir itself also affects the efficacy of numerous other medications, making it difficult to know how to administer them concurrently. In addition it can cause a large number of side-effects on its own.

Hence, providing brecanavir-based therapy without the need to co-administer ritonavir would be a desirable goal in that therapy is simplified and the side effects of ritonavir administration are eliminated.

HIV can never completely be eradicated so that persons infected with HIV pose a continuous risk of infecting others. After initial infection it takes a long time before the outbreak of the first symptoms of AIDS. People may live for years with the infection without experiencing any effects of it thereby being unaware of the risk of further transferring the virus to others. Prevention of HIV transmission therefore is crucial. Prevention currently focuses on avoiding transmission by sexual contacts, in particular by the use of condoms in populations at risk of being infected, on careful monitoring of blood samples for the presence of HIV and on avoiding of contact with blood of potentially infected subjects.

Despite these measures there is always an imminent risk of individuals being in contact with HIV infected persons of becoming infected. This in particular is the case for those providing medical care to infected patients or patients at risk of being infected such as physicians, nurses or dentists. Another group of individuals at risk are breast-fed infants whose mother is infected or at risk of becoming infected, especially in developing countries where alternatives for breast-feeding are less obvious.

Hence there is a need for further means that provide prevention against transmission of HIV. There is a particular need for effective prevention means that are easy to apply. Providing such prevention means is another object of the present invention.

It now has been found that the intermittent administration of parenteral formulations of brecanavir at time intervals of two weeks or longer, such as up to one year, results in plasma levels that are adequate in providing effective prevention of HIV infection or effective suppression of HIV infection. This allows for a reduced number of administrations thereby being beneficial in terms of pill burden and drug compliance of the patient. An additional advantage is that no additional agent that has a positive effect on drug metabolism and/or pharmacokinetics as to improve bioavailability, such as ritonavir, needs to be co-administered, thereby again reducing pill burden, as well as avoiding the side effects associated with the administration of ritonavir.

### Summary of the Invention

In one aspect the present invention relates to the use of a parenteral formulation comprising an anti-virally effective amount ofbrecanavir or a pharmaceutically acceptable acid-addition salt thereof, and a carrier, for the manufacture of a medicament for the prevention of transmission of HIV infection, or for the treatment of a subject infected with HIV, wherein the formulation is administered or is to be administered intermittently, by subcutaneous or intramuscular injection, at a time interval that is in the range of two weeks to one year.

In another aspect, there is provided a method of preventing the transmission of HIV infection, or of treating a subject infected with HIV, said method comprising the administration of a parenteral formulation comprising an anti-virally effective amount ofbrecanavir or a pharmaceutically acceptable acid-addition salt thereof, and a carrier, wherein the formulation is administered intermittently, by subcutaneous or intramuscular injection, at a time interval that is in the range of two weeks to one year.

In one embodiment the invention concerns a use or a method as specified herein, wherein the parenteral formulation is administered or is to be administered at a time interval that is in the range of two weeks to one month, or in the range of one month to three months, or in the range of three months to six months, or in the range of six months to twelve months.

In another embodiment the invention concerns a use or a method as specified herein, wherein the parenteral formulation is administered or is to be administered once every two weeks, or once every month, or once every three months.

One aspect of the present invention concerns the fact that the parenteral formulations can be administered without an agent that has a positive effect on drug metabolism and/or pharmacokinetics as to improve bioavailability, namely ritonavir.

In a further embodiment, the present invention is concerned with a pharmaceutical composition for administration by intramuscular or subcutaneous injection, comprising a therapeutically effective amount ofbrecanavir or a salt thereof, in the form of a suspension of micro- or nanoparticles comprising:
(a) brecanavir or a salt in micro- or nanoparticle form, having a surface modifier adsorbed to the surface thereof; and
(b) a pharmaceutically acceptable aqueous carrier; wherein the brecanavir active ingredient is suspended.

The invention further concerns a method of treating a subject infected with HIV, said method comprising the administration, by intramuscular or subcutaneous injection, of an anti-HIV effective amount pharmaceutical composition as specified above or hereinafter. Or, alternatively, the invention concerns the use of a pharmaceutical composition as specified above or hereinafter, for the manufacture of a medicament for treating HIV infection. In one embodiment, the composition is for the long-term treatment of I-IIV infection.

In another aspect, there is provided a method for the long term treatment of a subject infected with HIV, said method comprising the administration of an effective amount of a pharmaceutical composition as specified above or hereinafter, for administration by intramuscular or subcutaneous injection; wherein the composition is administered or is to be administered intermittently at a time interval that is in the range of two weeks to one year, or two weeks to one year. Or, alternatively, the invention concerns the use of a pharmaceutical composition as specified above or hereinafter, for the manufacture of a medicament for the long term treatment of a subject infected with HIV, for administration by intramuscular or subcutaneous injection, wherein the composition is administered or is to be administered intermittently at a time interval that is in the range of two weeks to one year, or two weeks to two years.

The invention further concerns a method for the prevention of HIV infection in a subject at risk of being infected by HIV, said method comprising administering an amount, effective in preventing HIV infection, of a pharmaceutical composition as specified above or as further specified hereinafter, to said subject. Or alternatively, the invention concerns the use of a pharmaceutical composition as specified above or as further specified hereinafter for the manufacture of a medicament for the prevention of HIV infection in a subject at risk of being infected by HIV.

In another aspect the invention relates to a method for the long term prevention of HIV infection in a subject at risk of being infected by HIV, said method comprising administering to said subject an effective amount of a pharmaceutical composition as specified above or as further specified hereinafter, wherein the composition is administered or is to be administered intermittently at a time interval that is in the range of two weeks to one year, or two weeks to two years.

The present invention furthermore relates to the use of a pharmaceutical composition as specified above or as further specified hereinafter, for the manufacture of a medicament for the long term prevention for the long term prevention of HIV infection in a subject at risk of being infected by HIV, wherein the composition is administered or is to be administered intermittently at a time interval that is in the range of two weeks to one year or two weeks to two years.

In one embodiment the invention concerns a use or a method as specified herein, wherein the pharmaceutical composition is administered or is to be administered at a time interval that is in the range of two weeks to one month, or in the range of one month to three months, or in the range of three months to six months, or in the range of six months to twelve months.

In another embodiment the invention concerns a use or a method as specified herein, wherein the pharmaceutical composition is administered or is to be administered once every two weeks, or once every month, or once every two months, or once every three months.

Further pharmaceutical compositions, methods of treatment or prevention, as well as uses for the manufacture of medicaments based on these compositions will be described hereinafter.

### Description of the figures

- Figure 1:: Individual plasma concentration (ng/ml) versus time profiles following intravenous dosing of a solution of brecanavir in rats at 2 mg/kg.
- Figure 2:: Individual and mean plasma concentrations of brecanavir following single subcutaneous injection of a brecanavir solution to rats at 16 (Δ) and 56 mg/kg (○).
- Figure 3:: Individual plasma concentration-time profiles of brecanavir following single intramuscular (○) and subcutaneous (Δ) injection in rats of
A. brecanavir Tween20 microsuspension (62.5 mg/ml) at 50 mg/kg;
B. brecanavir Tween20 nanosuspension (100 mg/ml) at 50 mg/kg;
C. brecanavir F108 nanosuspension (100 mg/ml) at 50 mg/kg;
D. brecanavir Tween20 nanosuspension (200 mg/ml) at 50 mg/kg.
- Figure 4:: Individual plasma concentration-time profiles of brecanavir in rabbits, following a single intramuscular dose of 50 mg/aminal of a Tween20 nanosuspension (100 mg/ml).

### Further Description of the Invention

Brecanavir can be used in base form or as a pharmaceutically acceptable salt form, such as an acid addition salt form. The pharmaceutically acceptable addition salts are meant to comprise therapeutically active non-toxic salt forms. Whenever used herein, the term "brecanavir" refers to as well the free form as any pharmaceutically acceptable salt thereof.

The term "prevention of HIV infection" relates to the prevention or avoidance of a subject becoming infected with HIV. The source of infection can be various, a material containing HIV, in particular a body fluid that contains HIV such as blood or sperm, or another subject who is infected with HIV. Prevention of HIV infection relates to the prevention of the transmission of the virus from the material containing HIV or from the HIV infected individual to an uninfected person, or relates to the prevention of the virus from entering the body from an uninfected person. Transmission of the HIV virus can be by any known cause of HIV transfer such as by sexual transmission or by contact with blood of an infected subject, e.g. medical staff providing care to infected subjects. Transfer of HIV can also occur by contact with HIV infected blood, e.g. when handling blood samples or with blood transfusion. It can also be by contact with infected cells, e.g. when carrying out laboratory experiments with HIV infected cells.

The terms "treatment of HIV infection", "anti-HIV therapy", as well as similar terms, refer to a treatment by which the viral load of HIV (represented as the number of copies of viral RNA in a specified volume of serum) is reduced. The more effective the treatment, the lower the viral load. Preferably the viral load should be reduced to as low levels as possible, e.g. below about 200 copies/ml, in particular below about 100 copies/ml, more in particular below 50 copies/ml, if possible below the detection limit of the virus. Reductions of viral load of one, two or even three orders of magnitude (e.g. a reduction in the order of about 10 to about 10², or more, such as about 10³) are an indication of the effectiveness of the treatment. Another parameter to measure effectiveness of anti-HIV treatment is the CD4 count, which in normal adults ranges from about 500 to about 1500 cells per µl. Lowered CD4 counts are an indication of HIV infection and once below about 200 cells per µl, AIDS may develop. An increase of CD4 count, e.g. with about 50, 100, 200 or more cells per µl, is also an indication of the effectiveness of anti-HIV treatment. The CD4 count in particular should be increased to a level above about 200 cells per µl, or above about 350 cells per µl. Viral load or CD4 count, or both, can be used to diagnose the degree of HIV infection.

The terms "effective treatment of HIV" and similar terms refer to that treatment that lowers the viral load, or increases CD4 count, or both, as described above.

The term "treatment of HIV infection" also relates to the treatment of diseases associated with HIV infection, for example AIDS, or other conditions associated with HIV infection including thrombocytopenia, Kaposi's sarcoma and infection of the central nervous system characterized by progressive demyelisation, resulting in dementia and symptoms such as, progressive dysarthria, ataxia and disorientation, and further conditions where HIV infection has also been associated with, such as peripheral neuropathy, progressive generalized lymphadenopathy (PGL), and AIDS-related complex (ARC).

The term "effective prevention of HIV" and similar terms refer to that situation where there is a decrease in the relative number of newly infected subjects in a population in contact with a source of HIV infection such as a material containing HIV, or a HIV infected subject. Effective prevention can be measured, for example, by measuring in a mixed population of HIV infected and non-infected individuals, if there is a decrease of the relative number of newly infected individuals, when comparing non-infected individuals treated with a pharmaceutical composition of the invention, and non-treated non-infected individuals. This decrease can be measured by statistical analysis of the numbers of infected and non-infected individuals in a given population over time.

The term "effective amount" refers to such an amount of brecanavir that, upon administration, provides effective treatment of HIV. The term "effective blood plasma levels" refers to those blood plasma levels of brecanavir that result in a reduction of the viral load, in particular below the number of copies mentioned above. The term "effective amount" similarly refers to an amount of brecanavir that, upon administration, provides effective prevention of HIV.

The terms "therapeutically effective amount", "an amount, effective in preventing HIV infection", and similar terms, refer to amounts of the active ingredient brecanavir that result in efficacious blood plasma levels. With "efficacious blood plasma levels" is meant those blood plasma levels of the HIV inhibitor brecanavir that provide effective treatment or effective prevention of HIV infection.

The term "subject" in particular relates to a human being.

The use or methods of using the parenteral compositions of brecanavir in accordance with this invention may be for the treatment of HIV infection, or for the prevention of transmission of HIV infection, during a long period of time. In the instance of prevention, the compositions will be applied for as long as the risk of infection exists. Or where the risk exists during a limited period of time, application will be during the period when the risk of infection is present, for example, a term in the range of two to three weeks, or three to four weeks, or a term in the range of one to two months, or two to three months, or three to six months, or six months to 12 months, or 12 months to 24 months. For treatment of HIV infection, the parenteral compositions of brecanavir will be administered for a prolonged period of time, such as a year or several years.

By the expression "effective for at least about two weeks or longer", one means that the minimal value of the plasma concentration at the end of the dosing interval, or between two dosing intervals of the active ingredient, brecanavir, should be above a threshold value. In case of therapeutic application said threshold value is the lowest plasma level at which brecanavir provides effective treatment of HIV infection. In case of application in the prevention of HIV infection said threshold value is the lowest plasma level at which brecanavir is effective in preventing transmission of HIV infection.

The pharmaceutical compositions of the present invention can be administered at various time intervals. When used in the prevention of HIV infection, the pharmaceutical compositions of this invention can be administered only once or a limited number of times such as twice, three, four, five, or six times, or more. This may be recommendable where prevention is required during a limited period of time, such as the period during which there is a risk of infection.

The pharmaceutical compositions of the present invention can be administered at the time intervals mentioned above, such as at a time interval that is in the range of two weeks to one month, or in the range of one month to three months, or in the range of three months to six months, or in the range of six months to twelve months. In one embodiment, the pharmaceutical composition can be administered once every two weeks, or once every month, or once every three months. In another embodiment the time interval is in the range of one to two weeks, or two to three weeks, or three to four weeks, or the time interval is in the range of one to two months, or two to three months, or three to four months, or three to six months, or six months to twelve months, or twelve months to 24 months. The time interval may be at least two weeks, but may also be several weeks, e.g. two, three, four, five or six weeks, or at time intervals of one month, or of several months, e.g. two, three, four, five or six months or even longer, e.g. seven, eight, nine or twelve months. In one embodiment, the pharmaceutical compositions of the present invention are administered at a time interval of one, two or three months. These longer periods between each administration of the pharmaceutical compositions of the invention provide further improvements in terms of pill burden and compliance. To further improve compliance, patients can be instructed to take their medication at a certain day of the week, where the composition is administered on a weekly schedule, or at a certain day of the month in case of a monthly schedule.

The length of the time intervals between each administration of a composition of the present invention may vary. For example said time intervals may be selected in function of the blood plasma levels. The intervals may be shorter where the blood plasma levels of brecanavir are deemed too low, e.g. when these approach the minimum blood plasma level specified hereinafter. The intervals may be longer where the blood plasma levels of brecanavir are deemed too high. In one embodiment, the compositions of the invention are administered at equal time intervals. The compositions preferably are administered without any interjacent additional administrations, or with other words, the compositions may be administered at particular points in time separated from one another by a time period of varying or equal length, e.g. a time period of at least two weeks, or any other time period specified herein, during which no further brecanavir is administered. Having time intervals of the same length has the advantage that the administration schedule is simple, e.g. administration takes place at the same day in the week, or the same day in the month. Such administration schedule therefore involves limited "pill burden" thereby contributing beneficially to the patient's compliance to the prescribed dosing regimen.

The dose (or amount) of brecanavir administered, which is the amount of brecanavir in the parenteral formulation for use in the invention, is selected such that the blood plasma concentration of brecanavir is kept during a prolonged period of time above a minimum blood plasma level. The term "minimum blood plasma level" (Cₘᵢₙ) in the context of HIV treatment refers to the lowest efficacious blood plasma level, the latter being that blood plasma level of brecanavir that provides effective treatment of HIV, or in alternative wording, that blood plasma level of brecanavir that is effective in suppressing HIV. The plasma levels of brecanavir should be kept above these threshold blood plasma levels because at lower levels the drug will no longer be effective, thereby increasing the risk of mutations. In the instance of HIV prevention, the term "minimum blood plasma level" (or Cₘᵢₙ) refers to the lowest blood plasma level of brecanavir that provides effective prevention of transmission of HIV infection, this is the lowest blood plasma level that is effective in inhibiting said transmission. The dose of brecanavir administered also depends on the time interval at which it is administered. The dose will be higher where administrations are less frequent.

The dose of brecanavir administered, depends on the amount of brecanavir in the pharmaceutical compositions of the invention, or on the amount of a given composition that is administered. Where higher blood plasma levels are desired, either or both of a composition of higher brecanavir concentration, or more of a given composition, may be administered. This applies vice versa if lower plasma levels are desired. Also a combination of varying time intervals and varying dosing may be selected to attain certain desired blood plasma levels.

The dose (or amount) of brecanavir administered also depends on the frequency of the administrations (i.e. the time interval between each administration). Usually, the dose will be higher where administrations are less frequent. All these parameters can be used to direct the blood plasma levels to desired values.

The dosing regimen also depends on whether prevention or treatment of HIV infection is envisaged. In case of therapy, the dose of brecanavir administered or the frequency of dosing, or both, are selected so that the blood plasma concentration of brecanavir is kept above the minimum blood plasma level. In particular, the blood plasma level of brecanavir is kept at a level above a minimum blood plasma level of about 24 ng/ml, or above about 40 ng/ml, or above about 50 ng/ml, or above about 75 ng/ml. The blood plasma level of brecanavir may be kept above a minimum blood plasma level that is higher, for example above about 100 ng/ml, or above about 150 ng/ml, or above about 200 ng/ml. The minimum blood plasma level can be determined by multiplying the EC₅₀ value with a factor expressing protein binding and a safety margin, which can be set at about 10. The EC₅₀ value can be obtained in a test using wild-type HIV. It is also known from Antimicrobial Agents and Chemotherapy, Apr. 2007, pp. 1202-1208.

In one embodiment, the blood plasma level of brecanavir is kept within certain ranges, in particular ranges starting from a minimum blood plasma level selected from those mentioned above and ending at a higher blood plasma levels such as about 100 ng/ml, or about 200 ng/ml, or about 500 ng/ml, or about 1000 ng/ml. In one embodiment, the blood plasma level of brecanavir is kept within the range of about 5 to about 500 ng/ml, or about 10 ng/ml to about 200 ng/ml, or about 10 ng/ml to about 100 ng/ml, or about 10 ng/ml to about 50 ng/ml.

The plasma levels of brecanavir should be kept above the above-mentioned minimum blood plasma levels because at lower levels the virus may no longer be sufficiently suppressed so that it can multiply with the additional risk of the emergence of mutations.

In particular, in the instance of HIV prevention, the blood plasma level of brecanavir can be kept at a level above a minimum blood plasma level mentioned above in relation to therapy. However in prevention the blood plasma level of brecanavir can be kept at a lower level, for example at a level above about 1 ng/ml, or about 5 ng/ml, or about 10 ng/ml. The blood plasma levels of brecanavir should preferably be kept above these minimum blood plasma levels because at lower levels the drug may no longer be effective thereby increasing the risk of HIV transmission. Plasma levels of brecanavir may be kept at somewhat higher levels to have a safety margin. Such higher levels start from about 24 ng/ml or more. The blood plasma level of brecanavir can be kept at a level that is in the ranges mentioned above in relation to therapy, but where the lower limits include the blood plasma levels of about 4 ng/ml, or about 5 ng/ml, or about 8 ng/ml.

In certain instances it may be desirable to keep the plasma levels of brecanavir at relatively low levels, e.g. as close as possible to the minimum blood plasma levels specified herein. This will allow reducing the frequency of the administrations and/or the quantity of brecanavir administered with each administration. It will also allow avoiding undesirable side effects, which will contribute to the acceptance of the dosage forms in most of the targeted population groups who are healthy people at risk of being infected and therefore are less inclined to tolerate side effects. The plasma levels of brecanavir may be kept at relatively low levels in the instance of prevention.
In other instances it may be desirable to keep the plasma levels of brecanavir at relatively higher levels, for example where there is a high risk of infection and more frequent and/or higher doses are not an issue. In these instances the minimum blood plasma level may be equal to the lowest blood plasma level of brecanavir that provides effective treatment of HIV, such as the specific levels mentioned herein.

In the instance of prevention, the dose to be administered parenterally should be calculated on a basis of about 0.2 mg/day to about 50 mg/day, or 0.5 mg/day to about 50 mg/day, or of about 1 mg/day to about 10 mg/day, or about 2 mg/day to about 5 mg/day, e.g. about 3 mg/day. This corresponds to a weekly dose of about 1.5 mg to about 350 mg, in particular of about 3.5 mg to about 350 mg, in particular of about 7 mg to about 70 mg, or about 14 mg to about 35 mg, e.g. about 35 mg, or to a monthly dose of from 6 mg to about 3000 mg, in particular about 15 mg to about 1,500 mg, more in particular of about 30 mg to about 300 mg, or about 60 mg to about 150 mg, e.g. about 150 mg. Doses for other dosing regimens can readily be calculated by multiplying the daily dose with the number of days between each administration.

In the instance of therapy, the dose to be administered parenterally should be somewhat higher and should be calculated on a basis of about 1 mg/day to about 150 mg/day, or of about 2 mg/day to about 100 mg/day, or of about 5 mg/day to about 50 mg/day, or about 10 mg/day to about 25 mg/day, e.g. about 15 mg/day. The corresponding weekly or monthly doses can be calculated as set forth above. For applications in prevention, the doses may be lower although the same dosing as for therapeutic applications may be used.

It has been found that, once administered, the blood plasma levels of brecanavir are more or less stable, i.e. they fluctuate within limited margins. The blood plasma levels have been found to approach more or less a steady state mode or to approximate more or less a zero order release rate during a prolonged period of time. By "steady state" is meant the condition in which the amount of drug present in the blood plasma of a subject stays at more or less the same level over a prolonged period of time. The plasma levels of brecanavir generally do not show any drops below the minimum plasma level at which the drug is effective. The term "stays at more or less the same level" does not exclude that there can be small fluctuations of the plasma concentrations within an acceptable range, e.g. fluctuations within a range of about ± 30 %, or about ± 20 %, or about ± 10 %, or about ± 5 %.

The parenteral formulations of brecanavir are administered intermittently at a time interval of at least two weeks, or in particular at a time interval mentioned herein, meaning that the parenteral formulation is administered without any interjacent additional administrations of brecanavir. Or with other words, brecanavir is administered at particular points in time separated from one another by a time period of at least two weeks, or in particular at a time interval mentioned herein, during which no brecanavir is administered. Hence the administration schedule is simple, requiring few administrations, and therefore dramatically reduces the problem of "pill burden" faced with standard HIV medication. This in turn will improve the patient's compliance to the prescribed medication.

The parenteral formulations of brecanavir can be administered at time intervals mentioned above. In one embodiment the time interval is in the range of two to three weeks or three to four weeks. In another embodiment the time interval is in the range of one to two months, or two to three months, or three to four months. The time interval may be at least two weeks, but may also be several weeks, e.g. two, three, four, five or six weeks, or at time intervals of one month, or of several months, e.g. two, three, four, five or six months or even longer, e.g. seven, eight, nine or twelve months. In one embodiment, the parenteral formulation is administered at a time interval of one, two or three months.

These longer periods between each administration of the parenteral formulation consist in an even further improvement of "pill burden" and compliance. To further improve compliance, patients can be instructed to take their medication at a certain day of the week, where the formulation is administered on a weekly schedule, or at a certain day of the month in case of a monthly schedule. The time intervals between each administration of a parenteral formulation of brecanavir may vary. For example, the intervals may be shorter where the blood plasma levels of brecanavir are deemed too low, e.g. when these approach the minimum blood plasma level specified hereinafter. The intervals may be longer where the blood plasma levels of brecanavir are deemed too high. In one embodiment, the parenteral formulations of brecanavir are administered at the same time intervals, for example every two weeks, every month, or at every time interval mentioned herein. Having time intervals of the same length has the advantage that the administration is e.g. at the same day in the week, or the same day in the month, thereby contributing to compliance of the therapy.

Preferably the parenteral formulation is administered in a single administration, for example by one injection after a time interval of at least two weeks, e.g. by one injection every two weeks, or every month, or every three months.

The plasma concentrations of brecanavir may reach relatively high levels, without causing significant side effects but should not exceed a maximum plasma level (or Cₘₐₓ), which is the blood plasma level where brecanavir causes significant side effects. As used herein, the term 'significant side effects' means that the side effects are present in a relevant patient population to an extend that the side effects affect the patients' normal functioning. The Cₘₐₓ for brecanavir can be determined from the extrapolation of test data in cellular assays or from the evaluation of clinical testing and preferably should not exceed a value of about 1000 ng/ml.

The parenteral brecanavir formulations are administered by subcutaneous or intramuscular administration.

### Carriers

The present invention is based on the use of parenteral formulations of the active ingredient brecanavir and therefore the nature of the carrier will have to be selected such as to suit parenteral administration. The carrier will be liquid and may be oily but in most cases will be aqueous. In the latter instance, the carrier comprises sterile water, though other ingredients may be included. The carrier may also contain a co-solvent, for example an alcohol such as ethanol, propanol, ethylene glycol, propylene glycol, or a polymer acting as co-solvent such as polyethylene glycol (PEG) or polyethoxylated castor oil (Cremophor^{®}).

To enhance solubility of the active compound additional ingredients may be added to the parenteral formulations of brecanavir that have a solubility promoting effect such as solubilizers and surfactants, or ingredients being both a surfactant and solubilizer. Examples of such additional ingredients are cyclodextrins or cyclodextrin derivatives. Appropriate cyclodextrins are α-, β-, γ-cyclodextrins or ethers and mixed ethers thereof wherein one or more of the hydroxy groups of the anhydroglucose units of the cyclodextrin are substituted with C₁₋₆alkyl, particularly methyl, ethyl or isopropyl, e.g. randomly methylated β-CD; hydroxyC₁₋₆alkyl, particularly hydroxyethyl, hydroxylpropyl or hydroxybutyl; carboxyC₁₋₆alkyl, particularly carboxymethyl or carboxyethyl; C₁₋₆alkylcarbonyl, particularly acetyl. Especially noteworthy as complexants and/or solubilizers are β-CD, randomly methylated β-CD, 2,6-dimethyl-β-CD, 2-hydroxyethyl-β-CD, 2-hydroxyethyl-β-CD, 2-hydroxypropyl-β-CD and (2-carboxy-methoxy)propyl-β-CD, and in particular 2-hydroxypropyl-β-CD (2-HP-β-CD).

Other such ingredients with surfactant properties are poloxamers, which are polyoxyethylene, polyoxypropylene block copolymers that conform generally to the formula HO-[CH₂CH₂O] ₓ-[CH(CH₃)CH₂O]_{y}-[CH₂CH₂O]_{z}-H wherein x, y and z can have various values, available under the tradename Pluronic^{®}, e.g. Pluronic^{®} F108, corresponding to poloxamer 338 in which the average values of x, y and z are respectively 128, 54 and 128. Still other such ingredients are the α-tocopheryl polyethylene glycol succinates, in particular Vitamin E TGPS; the polyoxyethylene sorbitan fatty acid esters (also referred to as polysorbates), available under the tradename Tween^{®} e.g. Tween^{®} 80; the polyethylene glycols (PEGs) such as PEG 400.

The parenteral formulations of brecanavir may further comprise suspending agents and buffers and/or pH adjusting agents, and optionally preservatives and isotonizing agents. Particular ingredients may function as two or more of these agents simultaneously, e.g. behave like a preservative and a buffer, or behave like a buffer and an isotonizing agent.

Buffering and pH adjusting agents can be used in an amount sufficient to render the dispersion neutral to very slightly basic (up to pH 8.5), preferably in the pH range of 7 to 7.5. Particular buffers are the salts of week acids. Buffering and pH adjusting agents that can be added may be selected from tartaric acid, maleic acid, glycine, sodium lactate/lactic acid, ascorbic acid, sodium citrates/citric acid, sodium acetate/acetic acid, sodium bicarbonate/carbonic acid, sodium succinate/succinic acid, sodium benzoate/ benzoic acid, sodium phosphates, tris(hydroxymethyl)aminomethane, sodium bicarbonate/sodium carbonate, ammonium hydroxide, benzene sulfonic acid, benzoate sodium/acid, diethanolamine, glucono delta lactone, hydrochloric acid, hydrogen bromide, lysine, methanesulfonic acid, monoethanolamine, sodium hydroxide, tromethamine, gluconic, glyceric, gluratic, glutamic, ethylene diamine tetraacetic (EDTA), triethanolamine, including mixtures thereof.

Preservatives comprise antimicrobials and anti-oxidants which can be selected from the group consisting of benzoic acid, benzyl alcohol, butylated hydroxyanisole (BHA), butylated hydroxytoluene (BHT), chlorbutol, a gallate, a hydroxybenzoate, EDTA, phenol, chlorocresol, metacresol, benzethonium chloride, myristyl-γ-piccolinium chloride, phenylmercuric acetate and thimerosal. Radical scavengers include BHA, BHT, Vitamin E and ascorbyl palmitate, and mixtures thereof. Oxygen scavengers include sodium ascorbate, sodium sulfite, L-cysteine, acetylcysteine, methionine, thioglycerol, acetone sodium bisulfite, isoacorbic acid, hydroxypropyl cyclodextrin. Chelating agents include sodium citrate, sodium EDTA and malic acid.

Isotonizing agents are, for example, sodium chloride, dextrose, sucrose, fructose, trehalose, mannitol, glycerin, sorbitol, xylitol, lactose, sodium sulfate. The suspensions conveniently comprise from 0 to 10% (w/v), in particular 0 to 6% of isotonizing agent. Typical nonionic isotonifiers are preferred, in particular glycerin, as electrolytes may affect colloidal stability.

In one embodiment, the parenteral formulations of brecanavir for use in accordance with the present invention take the form of a solution comprising an effective amount of becanavir and an aqueous carrier. Preferably, a solubilizer or surfactant is added, in particular any of the solubilizers or surfactants mentioned above.

It additionally has been found that the physico-chemical properties ofbrecanavir allow for the manufacture of micro- or nanoparticle suspensions that can be used for the long-term treatment of HIV infection as well as in the long-term prevention of HIV infection.

The term "micro- or nanoparticles" refers to particles in the micrometer or nanometer range. The size of the particles should be below a maximum size above which administration by subcutaneous or intramuscular injection becomes impaired or even no longer is possible. Said maximum size depends for example on the limitations imposed by the needle diameter or by adverse reactions of the body to large particles, or both. In one embodiment, the pharmaceutical compositions of the invention comprise brecanavir in nanoparticle form.

The average effective particle size of the micro- or nanoparticles of the present invention may be below about 50 µm, or below about 20 µm, or below about 10 µm, or below about 1000 nm, or below about 500 nm, or below about 400 nm, or below about 300 nm, or below about 200 nm. The lower limit of the average effective particle size may be low, e.g. as low as about 100 nm or as low as about 50 nm. In one embodiment, the average effective particle size is in the range of about 50 nm to about 50 µm, or about 50 nm to about 20 µm, or about 50 nm to about 10 µm, or about 50 nm to about 1000 nm, about 50 nm to about 500 nm, or about 50 nm to about 400 nm, or about 50 nm to about 300 nm, or about 50 nm to about 250 nm, or about 100 nm to about 250 nm, or about 150 nm to about 220 nm, or 100 to 200 nm, or about 150 nm to about 200 nm, e.g. about 130 nm, or about 150 nm.

As used herein, the term average effective particle size has its conventional meaning as known to the person skilled in the art and can be measured by art-known particle size measuring techniques such as, for example, sedimentation field flow fractionation, photon correlation spectroscopy, laser diffraction or disk centrifugation. The average effective particle sizes mentioned herein may be related to volume distributions of the particles. In that instance, by "an effective average particle size of less than about 50 µm" it is meant that at least 50% of the volume of the particles has a particle size of less than the effective average of 50 µm, and the same applies to the other effective particle sizes mentioned. In a similar manner, the average effective particle sizes may be related to weight distributions of the particles but usually this will result in the same or about the same value for the average effective particle size.

The micro- or nanoparticle compositions of the present invention provide release of the active ingredient brecanavir over a prolonged period of time. After administration, these compositions stay in the body and steadily release brecanavir, keeping this active ingredient in the patient's system for a prolonged period of time, thereby providing, during said period, anti-HIV therapy or prevention of transmission of HIV infection.

The micro- or nanoparticle compositions of the invention show good local tolerance and ease of administration. Good local tolerance relates to minimal irritation and inflammation at the site of injection; ease of administration refers to the size of needle and length of time required to administer a dose of a particular drug formulation. In addition, they show good stability and have an acceptable shelf life.

The micro- or nanoparticles of the present invention have a surface modifier adsorbed on the surface thereof. The function of the surface modifier is to act as a wetting agent as well as a stabilizer of the colloidial suspension.

In one embodiment, the micro- or nanoparticles in the compositions of the invention mainly comprise crystalline brecanavir or a salt thereof; and the surface modifier, the combined amount of which may at least comprise about 50%, or at least about 80%, or at least about 90%, or at least about 95%, or at least about 99% of the micro- or nano particles.

In a further aspect, the present invention is concerned with a pharmaceutical composition for administration by intramuscular or subcutaneous injection, comprising a therapeutically effective amount of brecanavir, or a stereoisomer or a stereoisomeric mixture thereof, in the form of a suspension of particles consisting essentially of:
(1) brecanavir, or a stereoisomer or a stereoisomeric mixture thereof in micro- or nanoparticle form, having a surface modifier adsorbed to the surface thereof; and
(2) a pharmaceutically acceptable aqueous carrier; wherein the active ingredient is suspended.

Suitable surface modifiers can be selected from known organic and inorganic pharmaceutical excipients, including various polymers, low molecular weight oligomers, natural products and surfactants. Particular surface modifiers include nonionic and anionic surfactants. Representative examples of surface modifiers include gelatin, casein, lecithin, salts of negatively charged phospholipids or the acid form thereof (such as phosphatidyl glycerol, phosphatidyl inosite, phosphatidyl serine, phosphatic acid, and their salts such as alkali metal salts, e.g. their sodium salts, for example egg phosphatidyl glycerol sodium, such as the product available under the tradename Lipoid^{™} EPG), gum acacia, stearic acid, benzalkonium chloride, polyoxyethylene alkyl ethers, e.g., macrogol ethers such as cetomacrogol 1000, polyoxyethylene castor oil derivatives; polyoxyethylene stearates, colloidal silicon dioxide, sodium dodecylsulfate, carboxymethylcellulose sodium, bile salts such as sodium taurocholate, sodium desoxytaurocholate, sodium desoxycholate; methylcellulose, hydroxyethylcellulose, hydroxypropylcellulose, hydroxypropylmethylcellulose, magnesium aluminate silicate, polyvinyl alcohol (PVA), poloxamers, such as Pluronic^{™} F68, F108 and F127 which are block copolymers of ethylene oxide and propylene oxide; tyloxapol; Vitamin E-TGPS (α-tocopheryl polyethylene glycol succinate, in particular α-tocopheryl polyethylene glycol 1000 succinate); poloxamines, such as Tetronic^{™} 908 (T908) which is a tetrafunctional block copolymer derived from sequential addition of ethylene oxide and propylene oxide to ethylenediamine; dextran; lecithin; dioctyl ester of sodium sulfosuccinic acid such as the products sold under the tradename Aerosol OT^{™} (AOT); sodium lauryl sulfate (Duponol^{™} P); alkyl aryl polyether sulfonate available under the tradename Triton^{™} X-200; polyoxyethylene sorbitan fatty acid esters (Tweens^{™} 20, 40, 60 and 80); sorbitan esters of fatty acids (Span^{™} 20, 40, 60 and 80 or Arlacel^{™} 20, 40, 60 and 80); polyethylene glycols (such as those sold under the tradename Carbowax^{™} 3550 and 934); sucrose stearate and sucrose distearate mixtures such as the product available under the tradename Crodesta^{™} F110 or Crodesta^{™} SL-40; hexyldecyl trimethyl ammonium chloride (CTAC); polyvinylpyrrolidone (PVP). If desired, two or more surface modifiers can be used in combination.

Particular surface modifiers are selected from poloxamers, α-tocopheryl polyethylene glycol succinates, polyoxyethylene sorbitan fatty acid esters, and salts of negatively charged phospholipids or the acid form thereof. More in particular the surface modifiers are selected from Pluronic^{™} F108 , Vitamin E TGPS, Tween^{™} 80, Tween^{™} 20, and Lipoid^{™} EPG. One or more of these surface modifiers may be used. Pluronic^{™} F108 corresponds to poloxamer 338 and is the polyoxyethylene, polyoxypropylene block copolymer that conforms generally to the formula HO-[CH₂CH₂O]ₓ-[CH(CH₃)CH₂O]_{y}-[CH₂CH₂O]_{z}-H in which the average values of x, y and z are respectively 128, 54 and 128. Other commercial names of poloxamer 338 are Hodag Nonionic^{™} 1108-F and Synperonic^{™} PE/F108. In one embodiment, the surface modifier comprises a combination of a polyoxyethylene sorbitan fatty acid ester and a phosphatidyl glycerol salt (in particular egg phosphatidyl glycerol sodium).

The optimal relative amount of brecanavir in relation to the surface modifier depends on the surface modifier selected, the specific surface area of the brecanavir suspension which is determined by the average effective particle size and the brecanavir concentration, the critical micelle concentration of the surface modifier if it forms micelles, etc. The relative amount (w/w) of brecanavir to the surface modifier preferably is in the range of 1 : 2 to about 20 : 1, in particular in the range of 1 : 1 to about 10:1, e.g. about 4 : 1.

The particles can be prepared by means of micronization/particle size reduction/nanonization by mechanical means and by controlled precipitation from a supersaturated solution, or by using supercritical fluids such as in the GAS technique ("gas anti-solvent"), or any combination of such techniques. A method is used comprising the steps of dispersing brecanavir in a liquid dispersion medium and applying mechanical means in the presence of grinding media to reduce the particle size of brecanavir to an average effective particle size of less than about 50 µm, in particular less than about 1,000 nm. The particles can be reduced in size in the presence of a surface modifier.

A general procedure for preparing the particles comprises
(a) obtaining brecanavir in microparticulated form, or if desired obtaining microparticulated brecanavir in micronized form;
(b) adding the brecanavir to a liquid medium to form a premix/predispersion; and
(c) subjecting the premix/predispersion to mechanical means in the presence of a grinding medium to reduce the average effective particle size.

The brecanavir starting material should be brought into microparticulated form, meaning that its average effective particle size is in the micrometer range. It is preferred that the average effective particle size of the brecanavir active agent for preparing the predispersion is less than about 100 µm as determined by sieve analysis. Where the average effective particle size of the micronized brecanavir is greater than about 100 µm, it is preferred that the particles of brecanavir be reduced in size to less than 100 µm by micronization using techniques known in the art.

The brecanavir can then be added to a liquid medium in which it is essentially insoluble to form a predispersion. The concentration of brecanavir in the liquid medium (weight by weight percentage) can vary widely and depends on the selected surface modifier and other factors. Suitable concentrations of brecanavir in compositions vary between about 0.1 % to about 60%, or between about 1% to about 60%, or between about 10% to about 50%, or between about 10% to about 30%, e.g. about 10%, 20% or 30% (each % in this paragraph relating to w/v).

The premix can be used directly by subjecting it to mechanical means to reduce the effective average effective particle size in the dispersion to less than 2,000 nm. The premix can be used directly when a ball mill is used for attrition. Alternatively, brecanavir and, optionally, the surface modifier, can be dispersed in the liquid medium using suitable agitation such as, for example, a roller mill, until a homogeneous dispersion is achieved.

The mechanical means applied to reduce the average effective particle size of brecanavir conveniently can take the form of a dispersion mill. Suitable dispersion mills include a ball mill, an attritor/attrition mill, a vibratory mill, a planetary mill, media mills, such as a sand mill and a bead mill. A media mill is preferred due to the relatively shorter milling time required to provide the desired reduction in particle size.

The grinding media for the particle size reduction step can be selected from rigid media preferably spherical or particulate in form having an average size less than 3 mm and, more preferably, less than 1 mm (as low as 200 µm beads). Such media desirably can provide the particles of the invention with shorter processing times and impart less wear to the milling equipment. Examples of grinding media are ZrO₂ such as 95% ZrO₂ stabilized with magnesia or stabilized with yttrium, zirconium silicate, glass grinding media, polymeric beads, stainless steel, titania, alumina and the like. Preferred grinding media have a density greater than 2.5 g/cm³ and include 95% ZrO₂ stabilized with magnesia and polymeric beads. The grinding media preferably are ZrO₂ beads.

The particles should be reduced in size at a temperature that does not significantly degrade the brecanavir compound. Processing temperatures of less than 30 to 40°C, in particular room temperature, are ordinarily preferred.

The pharmaceutical compositions according to the present invention contain an aqueous carrier that preferably is pharmaceutically acceptable. Said aqueous carrier comprises sterile water optionally in a mixture with other pharmaceutically acceptable ingredients. The latter comprise any ingredients for use in injectable formulations. These ingredients may be selected from one or more of a suspending agent, a buffer, a pH adjusting agent, a preservative, an isotonizing agent, and the like ingredients. In one embodiment, said ingredients are selected from one or more of a suspending agent, a buffer, a pH adjusting agent, and optionally, a preservative and an isotonizing agent. Particular ingredients may function as two or more of these agents simultaneously, e.g. behave like a preservative and a buffer, or behave like a buffer and an isotonizing agent.

Suitable buffering agents and pH adjusting agents should be used in amount sufficient to render the dispersion neutral to very slightly basic (up to pH 8.5), preferably in the pH range of 7 to 7.5. Particular buffers are the salts of week acids. Buffering and pH adjusting agents that can be added may be selected from tartaric acid, maleic acid, glycine, sodium lactate/lactic acid, ascorbic acid, sodium citrates/citric acid, sodium acetate/acetic acid, sodium bicarbonate/carbonic acid, sodium succinate/succinic acid, sodium benzoate/benzoic acid, sodium phosphates, tris(hydroxymethyl)aminomethane, sodium bicarbonate/sodium carbonate, ammonium hydroxide, benzene sulfonic acid, benzoate sodium/acid, diethanolamine, glucono delta lactone, hydrochloric acid, hydrogen bromide, lysine, methanesulfonic acid, monoethanolamine, sodium hydroxide, tromethamine, gluconic, glyceric, gluratic, glutamic, ethylene diamine tetraacetic (EDTA), triethanolamine, including mixtures thereof

Preservatives comprise antimicrobials and anti-oxidants which can be selected from the group consisting of benzoic acid, benzyl alcohol, butylated hydroxyanisole (BHA), butylated hydroxytoluene (BHT), chlorbutol, a gallate, a hydroxybenzoate, EDTA, phenol, chlorocresol, metacresol, benzethonium chloride, myristyl-γ-piccolinium chloride, phenylmercuric acetate and thimerosal. Radical scavengers include BHA, BHT, Vitamin E and ascorbyl palmitate, and mixtures thereof. Oxygen scavengers include sodium ascorbate, sodium sulfite, L-cysteine, acetylcysteine, methionine, thioglycerol, acetone sodium bisulfite, isoacorbic acid, hydroxypropyl cyclodextrin. Chelating agents include sodium citrate, sodium EDTA and malic acid.

An isotonizing agent or isotonifier may be present to ensure isotonicity of the pharmaceutical compositions of the present invention, and includes sugars such as glucose, dextrose, sucrose, fructose, trehalose, lactose; polyhydric sugar alcohols, preferably trihydric or higher sugar alcohols, such as glycerin, erythritol, arabitol, xylitol, sorbitol and mannitol. Alternatively, sodium chloride, sodium sulfate, or other appropriate inorganic salts may be used to render the solutions isotonic. These isotonifiers can be used alone or in combination. The suspensions conveniently comprise from 0 to 10% (w/v), in particular 0 to 6% of isotonizing agent. Of interest are nonionic isotonifiers, e.g. glucose, as electrolytes may affect colloidal stability.

A desirable feature for a pharmaceutical composition of the invention relates to the ease of administration. The viscosity of the pharmaceutical compositions of the invention should be sufficiently low to allow administration by injection. In particular they should be designed so that they can be taken up easily in a syringe (e.g. from a vial), injected through a fine needle (e.g. a 20 Gauge (G) 1½, 21 Gauge (G) 1½, 22 Gauge (G) 2 or 22 Gauge (G) 1½ needle) in not too long a time span. In one embodiment the viscosity of the compositions of the invention is below about 75 mPa·s, or below 60 mPa·s. Aqueous suspensions of such viscosity or lower usually meet the above-mentioned criteria.

Ideally, the aqueous suspensions according to the present invention will comprise as much brecanavir as can be tolerated so as to keep the injected volume to a minimum, in particular from 3 to 40% (w/v), or from 3 to 30% (w/v), or from 3 to 20% (w/v), or from 10 to 30% (w/v), of brecanavir. In one embodiment the aqueous suspensions of the invention contain about 10% (w/v) of brecanavir, or about 20% (w/v) of brecanavir, or about 30% (w/v) of brecanavir.

In one embodiment, the aqueous suspensions may comprise by weight, based on the total volume of the composition:
(a) from 3% to 50% (w/v), or from 10% to 40% (w/v), or from 10% to 30% (w/v), of brecanavir;
(b) from 0.5% to 10 %, or from 0.5% to 2% (w/v) of a wetting agent;
(c) from 0% to 10%, or from 0% to 5%, or from 0% to 2%, or from 0% to 1% of one or more buffering agents;
(d) from 0% to 10 %, or from 0% to 6% (w/v) of a isotonizing agent
(e) from 0% to 2% (w/v) preservatives; and
(f) water for injection q.s. ad 100%.

To the suspensions may optionally be added an amount of acid or base to bring the pH to a value of about pH 7. Suitable acids or bases are any of those that are physiologically acceptable, e.g. HCl, HBr, sulfuric acid, alkali metal hydroxides such as NaOH.

The administration of brecanavir as in the present invention may suffice to treat HIV infection although in a number of cases it may be recommendable to co-administer other HIV inhibitors. The latter preferably include HIV inhibitors of other classes, in particular those selected from NRTIs, NNRTIs and fusion inhibitors. In one embodiment, the other HIV inhibitor that is co-administered is an NNRTI. HIV inhibitors that may be co-administered by preference are those used in HAART combinations comprising a PI . For example two further NRTIs or an NRTI and a NNRTI may be co-administered. Such co-administration may be by oral administration or parenterally.

The present invention also concerns a pharmaceutical composition as described herein before for use as a medicament in the treatment or prophylaxis of HIV infection.

In addition, the present invention concerns the use of a pharmaceutical composition as described herein for the preparation of a medicament for the prophylaxis or treatment of HIV infection.

The present invention further concerns a method of treating a subject infected with HIV, said method comprising the administration of a therapeutically effective amount of a pharmaceutical composition as described herein.

As used herein, the word "substantially" does not exclude "completely" e.g. a composition which is "substantially free" from Y may be completely free from Y. Where necessary, the word "substantially" may be omitted from the definition of the invention. The term "about" in connection with a numerical value is meant to have its usual meaning in the context of the numerical value. Where necessary the word "about" may be replaced by the numerical value ±10%, or ±5%, or ±2%, or ±1%.
All documents cited herein are incorporated by reference in their entirety.

The following examples are intended to illustrate the present invention and should not be construed as limiting the invention thereto.

### Example

This example shows a study aimed at demonstrating that the administration of a parenteral formulation of brecanavir results in stable blood plasma levels during a prolonged period of time. By increasing the dose of brecanavir of the parenteral formulation higher blood plasma levels can be obtained.

Pharmacokinetic studies in rats were performed with parenteral (intravenous, intramuscular and subcutaneous) administration of brecanavir as a solution and brecanavir as an aqueous nano- or microsuspension to study the plasma kinetics at different doses.

### Intravenous injection of brecanavir solution

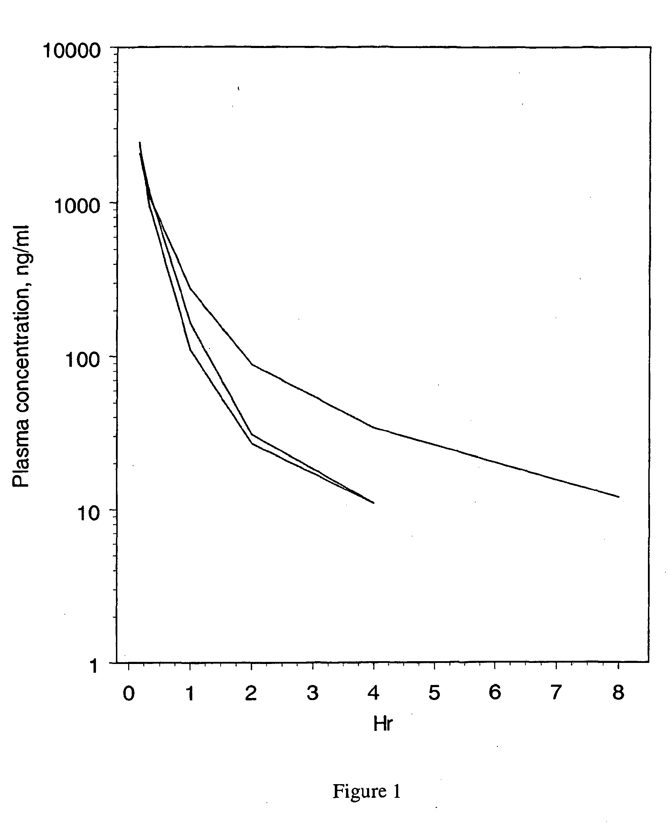

Brecanavir was injected intraveneously as a 1 mg/ml solution (PEG400/saline 70:30) in rats (n=3) to determine the plasma kinetics ofbrecanavir in these animals. Plasma samples were collected from each of the rats at selected time points after administration; samples were analyzed for brecanavir concentrations with LC/MS. (See Figure 1.)

Following i.v. injection of brecanavir the compound showed high concentrations and hence a moderate to small distribution volume. The compound was effectively cleared from the plasma with a moderate plasma clearance.

**Table 1: Pharmacokinetic parameters of brecanavir following i.v. injection of a solution in rats (n=3)**

| | | Rat 1 | Rat 2 | Rat 3 | Mean |
|---|---|---|---|---|---|
| AUCₗₐₛₜ | ng.h/ml | 1374 | 1066 | 1149 | 1196 |
| Cl | L/h/kg | 1.4 | 1.8 | 1.7 | 1.7 |
| Vc | L/kg | 0.70 | 0.46 | 0.52 | 0.56 |
| t_{1/2β} | h | 2.3 | 1.5 | 1.5 | 1.8 |

### Subcutaneous injection of brecanavir solution

Sprague-Dawley rats (Crl:CD^{®}(SD)IGS) of 200-300 g were injected once subcutaneously with an aqueous solution of brecanavir at 16 (n=4) and at 56 (n=4) mg/kg. The solution was prepared one day before dose administration; brecanavir was formulated in an aqueous 30 % (w/v) DMA/50 % (w/v) PEG400 solution at 35 mg/ml. The ingredients of the solution were: brecanavir, DMA 30 % (w/v), PEG 400 50 % (w/v) and pyrogenic free water. The content of brecanavir in the formulation was checked using LC-UV. The concentration of brecanavir in the formulation was 35 mg/ml.

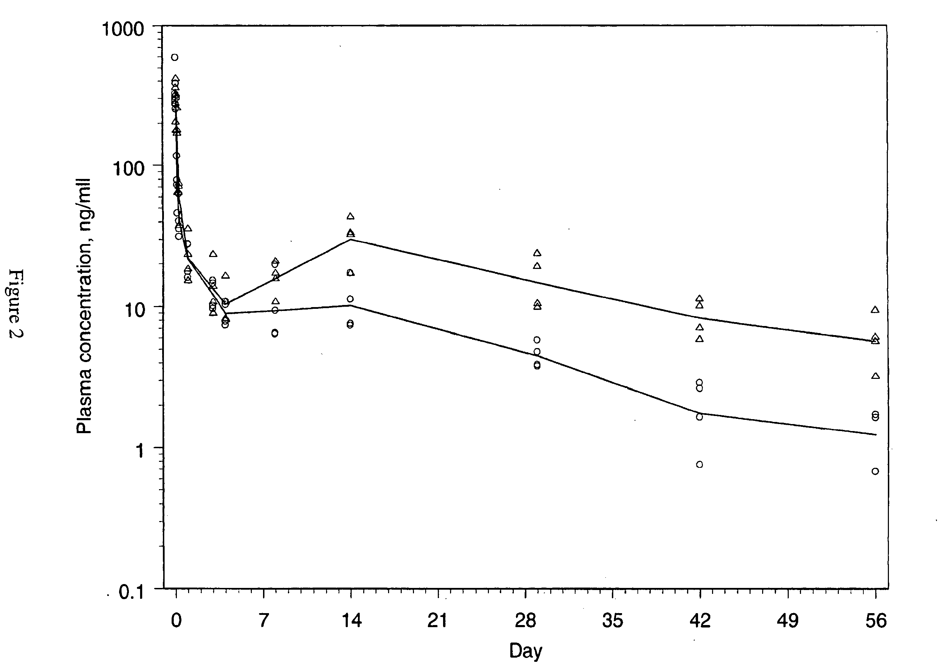

Blood samples (0.3 ml on EDTA) were collected from the rats via the tail vein at 1, 2, 4, 7 and 24 h and further at 3, 4, 8, 14, 29, 42 and 56 days after dosing. Plasma samples were analyzed for brecanavir with a qualified research method on LC/MS/MS. Plasma concentrations were plotted as a function of time after dosing (Figure 2). Pharmacokinetic parameters were calculated using WinNonlin software. Subcutaneous injection of an aqueous solution of brecanavir in rats produced an extended release pharmacokinetic profile with duration of at least 8 weeks (Figure 2). From the plasma concentration-time profile and the comparison of half-life values between i.v. and s.c. administration, it is clear that brecanavir following subcutaneous injection of a high dose solution shows a very slow absorption of the compound into the systemic circulation. The average absolute bioavailability of the 16 mg/kg dose was virtually complete (F=0.91), but somewhat lower for the higher dose, 56 mg/kg (F=0.66).

**Table 2: Pharmacokinetic parameters of brecanavir following a single s.c. injection of a brecanavir solution in rats (n=4) at 16 and 56 mg/kg.**

| 16 mg/kg | | | | | | |
|---|---|---|---|---|---|---|
| | | Rat 1 | Rat 2 | Rat3 | Rat 4 | Mean |
| Cₘₐₓ | ng/ml | 273 | 289 | 591 | 234 | 369 |
| t_{1/2λz} | h | 262 | 134 | 536 | 409 | 335 |
| AUCₗₐₛₜ | ng.h/ml | 6505 | 9630 | 9291 | 9448 | 8719 |

| 56 mg/kg | | | | | | |
|---|---|---|---|---|---|---|
| | | Rat 1 | Rat 2 | Rat3 | Rat 4 | Mean |
| Cₘₐₓ | ng/ml | 360 | 204 | 417 | 305 | 322 |
| t_{1/2λz} | h | 1272 | 388 | 460 | 1072 | 798 |
| AUCₗₐₛₜ | ng.h/ml | 29890 | 17602 | 24284 | 15221 | 21749 |

### Intramuscular and subcutaneous injections of brecanavir nano- and microsuspensions

Brecanavir was formulated as an aqueous nanosuspension containing 25 mg/ml of polysorbate 20 parenteral and 100 mg/ml of brecanavir. The ingredients of the nanosuspension were: brecanavir, polysorbate 20 parenteral 2.5 % (w/v), dextrose 5 % (w/v) and pyrogenic free water. The content of brecanavir in the formulation was checked using LC-UV. The concentration of brecanavir in the formulation was 100 mg/ml. The particle size distribution was measured on a Malvern Mastersizer^{™} using laser diffraction. The d₅₀ value was 125 nm.

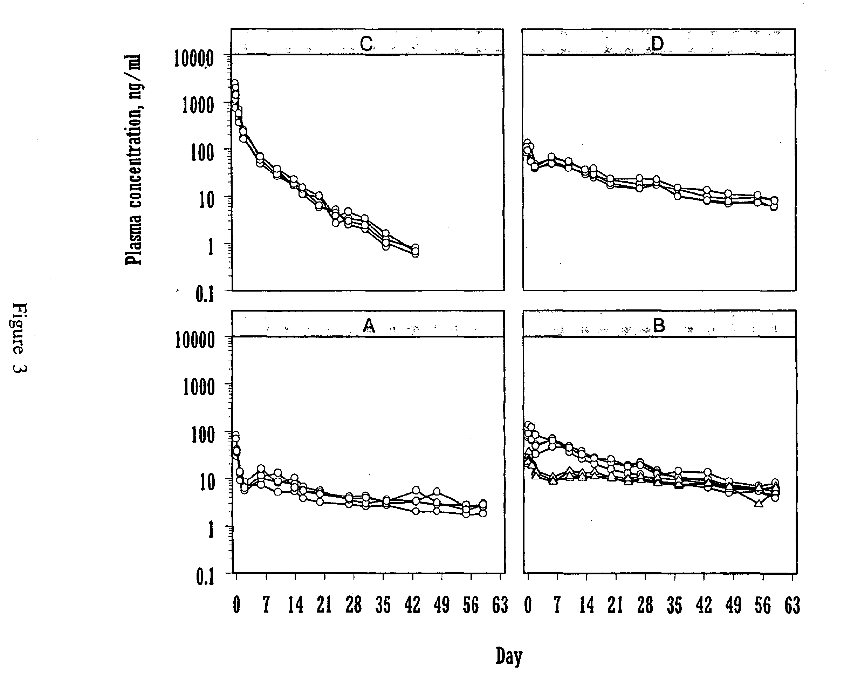

Sprague-Dawley rats (Crl:CD^{®}(SD)IGS) of 200-300 g were injected once with an aqueous nanosuspension of brecanavir at 50 mg/kg via the intramuscular (n=4) and the subcutaneous (n=4) route. Blood samples were collected from the tail vain at 1, 7 and 24 hours and further at 2, 6, 10, 13, 16, 20, 24, 27, 31, 36, 43, 48, 55 and 59 days after dosing. Plasma was separated and analyzed for brecanavir with a qualified research method on LC/MS/MS. Plasma concentrations were plotted as a function of time after dosing (Figure 3). Pharmacokinetic parameters were calculated using WinNonlin^{™} software.

The i.m. injection of the aqueous nanosuspension of brecanavir produced an extended release plasma concentration-time profile over a period of 56 days. The plasma concentrations following intramuscular injection were higher than those after subcutaneous injection. The amount released into the systemic circulation over this period is estimated to be complete (F ∼1) after intramuscular injection and approximately half (F ∼0.5) following subcutaneous injection. As compared to the subcutaneous injection of the solution, it was clear that s.c. injection of the nanosuspension produced a much lower peak plasma exposure after injection. Also, after the s.c. injection of the nanosuspension, plasma concentration remain very stable for a prolonged period of time at around 10 ng/ml, in contrast to the solution where plasma concentration continue to slowly decline after the initial 2 weeks.

Brecanavir was formulated as an microcontaining 25 mg/ml of polysorbate 20 parenteral and 100 mg/ml of brecanavir. The ingredients of the nanosuspension were: brecanavir, polysorbate 20 parenteral 2.5 % (w/v), dextrose 5 % (w/v) and pyrogenic free water. The content of brecanavir in the formulation was checked using LC-UV. The concentration of brecanavir in the formulation was 62.5 mg/ml. The particle size distribution was measured on a Malvern Mastersizer^{™} using laser diffraction. The d₅₀ value was 1.613 µm.

Sprague-Dawley rats (Crl:CD^{®}(SD)IGS) of 200-300 g were injected once with an aqueous nanosuspension of brecanavir at 50 mg/kg via the intramuscular (n=4). Blood samples were collected from the tail vain at 1, 7 and 24 hours and further at 2, 6, 10, 14, 16, 20, 24, 27, 31, 36, 43, 48, 55 and 59 days after dosing. Plasma was separated and analyzed for brecanavir with a qualified research method on LC/MS/MS. Plasma concentrations were plotted as a function of time after dosing (Figure 3). Pharmacokinetic parameters were calculated using WinNonlin^{™} software.

**Table 3: Pharmacokinetic parameters of brecanavir following a single i.m and s.c.. injection of a brecanavir nano- and microsuspension at 50 mg/kg.**

| Tween20 nanosuspension (100 mg/ml) i.m. | | | | | | |
|---|---|---|---|---|---|---|
| | | Rat 1 | Rat 2 | Rat3 | Rat 4 | Mean |
| Cₘₐₓ | ng/ml | 86.5 | 127 | 89.1 | 134 | 109 |
| AUCₗₐₛₜ | ng.h/ml | 29097 | 33753 | 26686 | 36728 | 31566 |

| Tween20 nanosuspension (100 mg/ml) s.c | | | | | | |
|---|---|---|---|---|---|---|
| | | Rat 1 | Rat 2 | Rat3 | Rat 4 | Mean |
| Cₘₐₓ | ng/ml | 30.2 | 32.3 | 32.4 | 37.0 | 33.0 |
| AUCₗₐₛₜ | ng.h/ml | 13644 | 12470 | 12478 | 13545 | 13034 |

| Tween20 microsuspension (62.5 mg/ml) i.m. | | | | | | |
|---|---|---|---|---|---|---|
| | | Rat 1 | Rat 2 | Rat3 | Rat 4 | Mean |
| Cₘₐₓ | ng/ml | 34.5 | 40.4 | 82.0 | 68.7 | 56.4 |
| AUCₗₐₛₜ | ng.h/ml | 5371 | 8416 | 7272 | 8056 | 7279 |

| F108 nanosuspension (100 mg/ml) i.m. | | | | | | |
|---|---|---|---|---|---|---|
| | | Rat 1 | Rat 2 | Rat3 | Rat 4 | Mean |
| Cₘₐₓ | ng/ml | 1580 | 2430 | 1120 | 1370 | 1625 |
| AUCₗₐₛₜ | ng.h/ml | 49007 | 67432 | 41803 | 56420 | 53666 |

| Tween20 nanosuspension (200 mg/ml) i.m. | | | | | | |
|---|---|---|---|---|---|---|
| | | Rat 1 | Rat 2 | Rat3 | Rat 4 | Mean |
| Cₘₐₓ | ng/ml | 107 | 95.7 | 132 | 106 | 110 |
| AUCₗₐₛₜ | ng.h/ml | 28988 | 31139 | 34090 | 38176 | 33098 |

### Intramuscular injection of brecanavir nanosuspension in rabbits

Brecanavir was formulated as an aqueous nanosuspension containing 25 mg/ml of polysorbate 20 parenteral and 100 mg/ml of brecanavir. The ingredients of the nanosuspension were: brecanavir, polysorbate 20 parenteral 2.5 % (w/v), dextrose 5 % (w/v) and pyrogenic free water. The content of brecanavir in the formulation was checked using LC-UV. The concentration of brecanavir in the formulation was 100 mg/ml. The particle size distribution was measured on a Malvern Mastersizer^{™} using laser diffraction. The d₅₀ value was 125 nm.

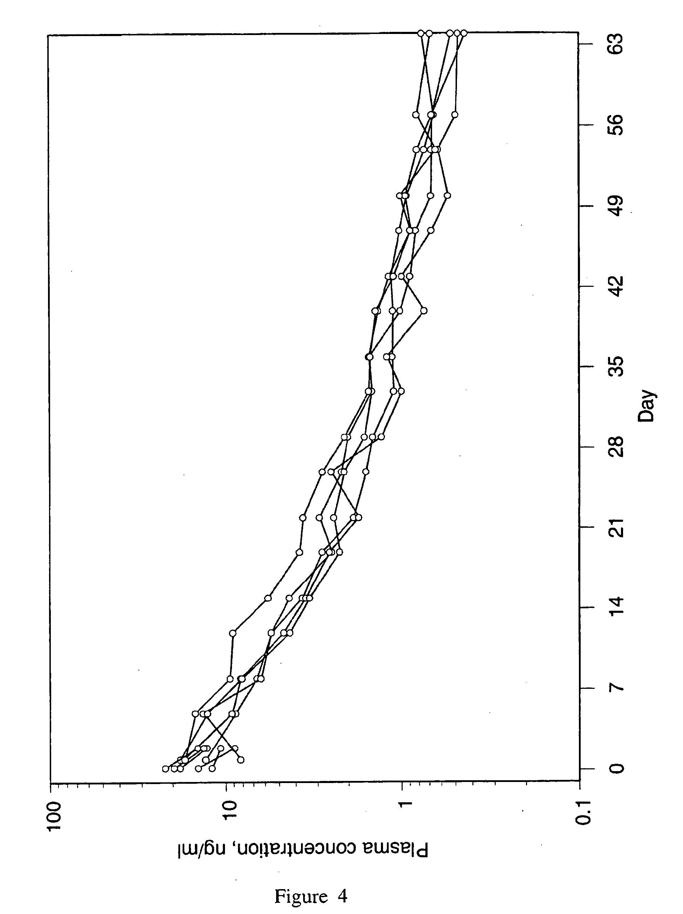

New Zealand White rabbits approx. 2.5 kg were injected once with an aqueous nanosuspension of brecanavir at 50 mg per rabbit via the intramuscular (n=5) route. Blood samples were collected from the tail vain at 6 and 24 hours and further at 2, 5, 8, 12, 15, 19, 22, 26, 29, 33, 36, 40, 43, 47, 50, 54, 57, 64 and 71 days after dosing. Plasma was separated and analyzed for brecanavir with a qualified research method on LC/MS/MS. Plasma concentrations were plotted as a function of time after dosing (Figure 4). Pharmacokinetic parameters were calculated using WinNonlin^{™} software.

**Table 4: Pharmacokinetic parameters of brecanavir in rabbits following a single i.m. injection with a Tween20 nanosuspension (100 mg/ml) of brecanavir in rabbits.**

| | | Tween20 nanosuspension (100 mg/ml) i.m. | | | | | |
|---|---|---|---|---|---|---|---|
| | | Rabbit 1 | Rabbit 2 | Rabbit3 | Rabbit 4 | Rabbit 5 | Mean |
| Cₘₐₓ | ng/ | 56.5 | 32.3 | 48.5 | 61.9 | 33.7 | 46.6 |
| AUCₗₐₛₜ | ng.h | 19089 | 14855 | 17388 | 19346 | 15045 | 17144 |

## Claims

1. The use of a parenteral formulation comprising an anti-virally effective amount of brecanavir or a pharmaceutically acceptable acid-addition salt thereof, and a carrier, without an additional agent that has a positive effect on drug metabolism and/or pharmacokinetics as to improve bioavailability wherein the additional agent is ritonavir for the manufacture of a medicament for the treatment of a subject infected with HIV, wherein the formulation is to be administered by subcutaneous or intramuscular injection intermittently at a time interval that is in the range of two weeks to one year.

2. The use according to claim 1, wherein the parenteral formulation is a solution comprising an effective amount of brecanavir and an aqueous carrier.

3. The use according to claim 2, wherein a solubilizer or surfactant is added to the said solution.

4. The use according to any of claims 1 - 3, wherein the formulation is to be administered at a time interval that is in the range of two weeks to one month.

5. The use according to any of claims 1 - 3, wherein the formulation is to be administered at a time interval that is in the range of one month to three months.

6. The use according to any of claims 1 - 3, wherein the formulation is to be administered at a time interval that is in the range of three months to six months.

7. The use according to any of claims 1 - 3, wherein the formulation is to be administered once every month.

8. The use according to any of claims 1 - 3, wherein the formulation is to be administered once every three months.

9. The use according to claim 1 wherein the blood plasma level is kept at a level above 28 ng/ml.

10. A pharmaceutical composition for administration by intramuscular or subcutaneous injection, comprising a therapeutically effective amount of brecanavir or a salt thereof, in the form of a suspension of micro- or nanoparticles comprising:
(a) brecanavir or a salt thereof, in micro- or nanoparticle form, having a surface modifier adsorbed to the surface thereof; and
(b) a pharmaceutically acceptable aqueous carrier; wherein the brecanavir active ingredient is suspended.

11. A composition according to claim 10, wherein the surface modifier is selected from the group of poloxamers, α-tocopheryl polyethylene glycol succinates, polyoxyethylene sorbitan fatty acid esters, and salts of negatively charged phospholipids.

12. A composition according to claims 10 or 11, wherein the surface modifier is selected frompoloxamer 338, Vitamin E-TGPS, polysorbate-80, polysorbate-20 and egg phosphatidyl glycerol sodium.

13. A composition according to any of claims 10 to 12, wherein the average effective particle size of brecanavir micro- or nanoparticles is below 200 nm.

14. The use according to any of claims 1 to 3, wherein the parenteral formulation is a pharmaceutical composition as defined in any of claims 10 to 13.

## Patentansprüche

1. Verwendung einer eine antiviralwirksame Menge an Brecanavir oder eines pharmazeutisch unbedenklichen Säureadditionssalzes davon und einen Träger ohne ein zusätzliches Mittel mit einer positiven Wirkung auf Arzneimittelmetabolisierung und/oder -pharmakokinetik zur Verbesserung der biologischen Verfügbarkeit, wobei es sich bei dem zusätzlichen Mittel um Ritonavir handelt, umfassenden parenteralen Formulierung zur Herstellung eines Medikaments zur Behandlung eines mit HIV infizierten Patienten, wobei die Formulierung durch periodische subkutane oder intramuskuläre Injektion in einem Zeitintervall im Bereich von 2 Wochen bis zu einem Jahr verabreicht wird.

2. Verwendung nach Anspruch 1, wobei es sich bei der parenteralen Formulierung um eine Lösung handelt, die eine wirksame Menge an Brecanavir und einen wässrigen Träger umfasst.

3. Verwendung nach Anspruch 2, wobei der Lösung ein Lösungsvermittler oder Tensid zugesetzt wird.

4. Verwendung nach einem der Ansprüche 1 - 3, wobei die Formulierung in einem Zeitintervall im Bereich von 2 Wochen bis einem Monat verabreicht wird.

5. Verwendung nach einem der Ansprüche 1 - 3, wobei die Formulierung in einem Zeitintervall im Bereich von einem Monat bis drei Monaten verabreicht wird.

6. Verwendung nach einem der Ansprüche 1 - 3, wobei die Formulierung in einem Zeitintervall im Bereich von drei Monaten bis sechs Monaten verabreicht wird.

7. Verwendung nach einem der Ansprüche 1 - 3, wobei die Formulierung einmal jeden Monat verabreicht wird.

8. Verwendung nach einem der Ansprüche 1 - 3, wobei die Formulierung einmal alle drei Monate verabreicht wird.

9. Verwendung nach Anspruch 1, wobei der Blutplasmaspiegel auf einem Spiegel über 28 ng/ml gehalten wird.

10. Pharmazeutische Zusammensetzung zur Verabreichung durch intramuskuläre oder subkutane Injektion, umfassend eine therapeutisch wirksame Menge an Brecanavir oder eines Salzes davon, in Form einer Suspension von Mikro- oder Nanopartikeln umfassend:
(a) Brecanavir oder ein Salz davon in Mikro- oder Nanopartikelform mit einer auf der Oberfläche davon adsorbierten oberflächenmodifizierenden Substanz und
(b) einen pharmazeutisch unbedenklichen wässrigen Träger, in dem der Brecanavir-Wirkstoff suspendiert ist.

11. Zusammensetzung nach Anspruch 10, wobei die oberflächenmodifizierende Substanz aus der Gruppe der Poloxamere, α-Tocopherylpolyethylenglykolsuccinate, Polyoxyethylensorbitanfettsäureester und Salze negativ geladener Phospholipide ausgewählt ist.

12. Zusammensetzung nach Anspruch 10 oder 11, wobei die oberflächenmodifizierende Substanz aus Poloxamer 338, Vitamin E-TGPS, Polysorbat-80, Polysorbat-20 und Eiphosphatidylglycerol-Natrium ausgewählt ist.

13. Zusammensetzung nach einem der Ansprüche 10 bis 12, wobei die durchschnittliche effektive Teilchengröße der Brecanavir-Mikro- oder -Nanopartikel unter 200 nm liegt.

14. Verwendung nach einem der Ansprüche 1 bis 3, wobei es sich bei der parenteralen Formulierung um eine wie in einem der Ansprüche 10 bis 13 definierte pharmazeutische Zusammensetzung handelt.

## Revendications

1. Emploi d'une formule parentérale comprenant une quantité antivirale active de brécanavir ou l'un de ses sels d'addition acide de qualité pharmaceutique, et un vecteur, sans agent supplémentaire exerçant un effet positif sur le métabolisme et/ou la pharmacocinétique du médicament de sorte à améliorer la biodisponibilité, l'agent supplémentaire étant le ritonavir, dans la fabrication d'un médicament destiné au traitement d'un sujet infecté par le VIH, la formule étant destinée à être administrée de façon intermittente par injection sous-cutanée ou intramusculaire dans un intervalle de temps de l'ordre de deux semaines à un an.

2. Emploi conforme à la revendication 1, où la formule parentérale est une solution comprenant une quantité active de brécanavir et un vecteur aqueux.

3. Emploi conforme à la revendication 2, où un agent de solubilisation ou un tensioactif est ajouté à ladite solution.

4. Emploi conforme à l'une quelconque des revendications 1 à 3, où la formule doit être administrée à des intervalles de temps de l'ordre de deux semaines à un mois.

5. Emploi conforme à l'une quelconque des revendications 1 à 3, où la formule doit être administrée à des intervalles de temps de l'ordre d'un mois à trois mois.

6. Emploi conforme à l'une quelconque des revendications 1 à 3, où la formule doit être administrée à des intervalles de temps de l'ordre de trois mois à six mois.

7. Emploi conforme à l'une quelconque des revendications 1 à 3, où la formule doit être administrée une fois par mois.

8. Emploi conforme à l'une quelconque des revendications 1 à 3, où la formule doit être administrée une fois tous les trois mois.

9. Emploi conforme à la revendication 1, où la concentration plasmatique est maintenue à un niveau supérieur à 28 ng/ml.

10. Composition pharmaceutique pour administration par injection intramusculaire ou sous-cutanée, comprenant une quantité thérapeutiquement active de brécanavir ou de l'un de ses sels, sous la forme d'une suspension de micro- ou de nanoparticules comprenant :
(a) le brécanavir ou l'un de ses sels, sous forme de micro- ou de nanoparticules, présentant un modificateur de surface absorbé à sa surface ; et
(b) un vecteur aqueux de qualité pharmaceutique ; dans lequel le principe actif brécanavir est suspendu.

11. Composition conforme à la revendication 10, où le modificateur de surface est choisi dans le groupe constitué par les suivants : poloxamères, succinates d'α-tocophéryl-polyéthylène glycol , esters d'acide gras de polyoxyéthylène sorbitane, et sels de phospholipides négativement chargés.

12. Composition conforme aux revendications 10 ou 11, où le modificateur de surface est choisi parmi le poloxamère 338, la Vitamine E-TGPS, le polysorbate-80, le polysorbate-20 et le phosphatidylglycérol sodium d'oeuf.

13. Composition conforme à l'une quelconque des revendications 10 à 12, où la granulométrie efficace moyenne des micro- ou des nanoparticules de brécanavir est inférieure à 200 nm.

14. Emploi conforme à l'une quelconque des revendications 1 à 3, où la formule parentérale est une composition pharmaceutique conforme à l'une quelconque des revendications 10 à 13.
